Europäisches Patentamt

European Patent Office

Office européen des brevets

(11) Publication number: **0 363 262**
**A1**

# EUROPEAN PATENT APPLICATION

(21) Application number: **89402692.1**

(22) Date of filing: **29.09.89**

(51) Int. Cl.5: **C12M 3/00**

(30) Priority: **30.09.88 JP 246177/88**

(43) Date of publication of application:
**11.04.90 Bulletin 90/15**

(84) Designated Contracting States:
**BE DE FR GB IT NL SE**

(71) Applicant: **Terumo Kabushiki Kaisha**
**No. 44-1, Hatagaya 2-chome Shibuya-ku**
**Tokyo 151(JP)**

(72) Inventor: **Katakura, Takeo**
**c/o Terumo K.K. 2656-1, Ohbuchi**
**Fuji-shi Shizuoka-ken(JP)**
Inventor: **Makino, Ayako**
**c/o Terumo K.K. 2656-1, Ohbuchi**
**Fuji-shi Shizuoka-ken(JP)**
Inventor: **Yagishita, Akira**
**c/o Terumo K.K. 2656-1, Ohbuchi**
**Fuji-shi Shizuoka-ken(JP)**
Inventor: **Ohsawa, Takaaki**
**c/o Terumo K.K. 2656-1, Ohbuchi**
**Fuji-shi Shizuoka-ken(JP)**
Inventor: **Fujii, Tatsuya**
**c/o Terumo K.K. 2656-1, Ohbuchi**
**Fuji-shi Shizuoka-ken(JP)**

(74) Representative: **Gillard, Marie-Louise et al**
**Cabinet Beau de Loménie 55, Rue**
**d'Amsterdam**
**F-75008 Paris(FR)**

(54) **Cell-incubator.**

(57) A cell-incubator comprising, a housing (1) which has a room defined by housing walls, an inlet port (9) and an outlet port (10) for the first medium, each of which is provided on the housing wall, an inlet port (11) and an outlet port (12) for the second medium, each of which is provided on the housing wall, a first medium fluid channel (5) which is formed in the room and communicates with the inlet port and outlet port for the first medium, a second medium fluid channel (6) which is formed in the room and communicates with the inlet port and outlet port for the second medium, a first substance exchanging membrane (2) which constitutes at least a part of a wall defining the first medium fluid channel, a second substance exchanging membrane (3) which constitutes at least a part of a wall defining the second medium fluid channel, and cell culture chamber (4) which is formed between the first medium fluid channel and the second medium fluid channel and at least a part of which is defined by the first and second substance exchanging membranes.

## Cell-incubator

The present invention relates to a cell-incubator, particularly to a cell-incubator which is suitable for cultivating cells of high adhesion dependency and blood cells. The invention also relates to an artificial liver produced by cultivating hepatocytes in the cell-incubator.

Mass culture of cells has been conventionally performed by suspension culture method or a culture on a surface of separation membrane. However, each of these methods suffers from the following problems.

For example, interstitial cells such as fibroblasts, smooth muscle cells, and cerebral cells, or high-functional cells such as hepatocytes, pancreatic cells, nerve cells, endothelial cells, and epithelial cells cannot be cultivated by suspension culture, because of their high adhesion dependency. Therefore, suspension culture of these high-functional cells has been conducted by making the cells to adhere on surfaces of beads which are called carriers. This method, however, leads to reduced cell activities due to stirring and aeration both of which are required in suspension culture. Moreover, since perfusion fluid is directly contacted with the cells, complicated procedures are necessary to separate the cells, when collecting useful metabolites from the fluid.

On the other hand, in culture on a surface of a separation membrane, hollow fibers are mainly used; cells are charged outside the fibers, and a culture solution is flowed inside the fibers. When oxygen requiring cells are cultured by use of hollow fibers, an oxygenation operation is necessary in combination. Moreover, for attaining a optimum cultivation, culture condition including temperature should be controlled precisely.

With regard to the cell-culture on a surface of a separation membrane, it has been reported that a substance exchanging device of hollow fiber type is utilized as an incubator to culture cells on the outer surface of the fibers (e.g. Knazek R.A., et al.: Cell Culture on Artificial Capillaries; An approach to tissue growth in vitro, SCIENCE, vol. 178,65, 1972). In this incubator of hollow fiber type, cells and a perfusion fluid are contacted with each other through only one membrane, thus the membrane is functionally restricted to transfer solutes only. Therefore, when performing gaseous concentration adjustment which is essential for cell culture, a large scale apparatus is necessary such that the overall incubator is mounted in a carbon dioxide incubator, or complicated procedures are necessary in order to precisely flow incubation gas into the culture solution by means of a dissolved oxygen controller (D0 controller), etc.

The object of the invention is to provide a cell-incubator which is suitable to cultivate high-functional cells of high adhesion dependency and oxygen requiring cells, and which easily gives suitable culture conditions.

This object can be attained by a cell-incubator comprising; a housing which has a room defined by housing walls, an inlet port and an outlet port for the first medium, each of which is provided on the housing wall, an inlet port and an outlet port for the second medium, each of which is provided on the housing wall, a first medium fluid channel which is formed in the room and communicates with the inlet port and outlet port for the first medium, a second medium fluid channel which is formed in the room and communicates with the inlet port and outlet port for the second medium, a first substance exchanging membrane which constitutes at least a part of a wall defining the first medium fluid channel, a second substance exchanging membrane which constitutes at least a part of a wall defining the second medium fluid channel, and cell culture chamber which is formed between the first medium fluid channel and the second medium fluid channel and at least a part of which is defined by the first and second substance exchanging membranes.

This invention can be more fully understood from the following detailed description when taken in conjunction with the accompanying drawings, in which:

Fig. 1 is a cross-sectional view of a cell-incubator according to one embodiment of the present invention;

Fig. 2 shows a method to utilize a cell-incubator according to the embodiment in Fig. 1;

Fig. 3 shows cell-culturing in a cell-incubator in Fig. 1; and

Fig. 4 shows a cell-incubator according to another embodiment of the invention.

Detailed description of the present invention is as follows.

As noted above, a cell-incubator comprises two fluid channels for the first and second mediums. These channels may be used for both liquid flow and gas flow.

A fluid channel for liquid medium may preferably be determined by a substance exchanging membrane which is porous membrane having pores of 5 Å - 10 μm in mean diameter. Examples of the membrane include a separating membrane which is formed of regenerated cellulose, cellulose acetate, polyacrylonitrile, polymethyl methacrylate, polysulfone, polyethylene, polypropylene, polyvinylidene fluoride, etc.. The membrane is preferably hydrophilic to obtain high-efficient solute permeability. When utilizing a hydrophobic polyolefin membrane, the membrane is preferably treated to obtain hydrophilicity.

On the other hand, examples of a substance exchanging membrane which determines the fluid channel for gaseous medium include a gas-exchanging membrane formed of polypropylene, silicone, polyethylene fluoride which is known under the tradename Teflon.

The invention may utilize a porous sponge sheet or a sponge mesh having open cells charged in a culture chamber. Collagen gels may be charged in the chamber together with or in place of the sponge.

A cell-incubator of the invention may be formed by stacking a plural of culture chambers. Between two culture chambers of this embodiment, either a first medium fluid channel or a second medium fluid channel is provided.

When cell-culture is performed by utilizing a cell-incubator of the invention, cells to be cultured are charged in a culture chamber, and each of given medium is charged in a first medium fluid channel and a second medium fluid channel. For example, a culture solution is flowed in a liquid fluid channel, and incubation gas mixture containing required gas ingredients is flowed in a gas fluid channel. Due to this construction, a fresh culture solution can be supplied from the liquid channel through the membrane into the culture chamber and waste products excreted from cultured cells are rapidly removed through the membranes. At the same time, required gas ingredients can be supplied from the gas channel through the membrane into the culture chamber. Thus the culture chamber can be maintained at optimum culture conditions.

Therefore, good cell-culturing can be obtained without either carbon dioxide gas incubator or a gas flowing machine, both of which have been conventionally required.

Moreover, when hepatocytes is cultured in a cell-incubator of the invention, the incubator which has been charged with cultured hepatocytes can be utilized as an artificial liver apparatus as it is. At this time, patient blood can be extracorporeally circulated through the first channel or the second channel. The following is description of this artificial liver apparatus.

Into the artifical liver apparatus, collagen gels are charged as well as hepatocytes. Effects of collagen gel are as follows;

(1) to enable high-density culture of hepatocytes, and thus make the artificial liver apparatus compacted.

(2) to maintain activities of the hepatocytes for a long period by means of enclosing the cells into the gel and stabilizing the cells. Particularly, when fibroblasts derived from liver, lung, and cardiac corium, mesenchyme cells such as vascular endothelial cells, etc. are enclosed together with the hepatocytes, excellent effects can be obtained to maintain the hepatocytes stable.

(3) Even if the substance exchanging membrane is destroyed, the hepatocytes can be avoided to diffuse into blood by enclosing the cells inside the gel.

On the other hand, the substance exchanging membrane functions as follows;

(1) to physically strengthen the fragile gel of high water-containing ratio without causing deterioration of reaction efficiency.

(2) to enable the fragile gel of high water-containing ratio to be molded into fine fiber shape or thin film shape. Therefore, surface area/volume of the gel can be increased to eminently promote reaction efficiency.

The artificial liver apparatus of the invention has such a construction that hepatocytes are charged in a porous membrane. Blood or blood plasma to be detoxicated are contacted with said hepatocytes by diffusion through the porous membrane and subjected to metabolism of the hepatocytes. Therefore, blood contamination with hepatocytes can be thoroughly cancelled, while the hepatocytes can be easily contacted with blood. Complicated procedures can be also avoided at use.

Next, preferred embodiments of a cell-incubator according to the present invention are described as follows with reference to the drawings.

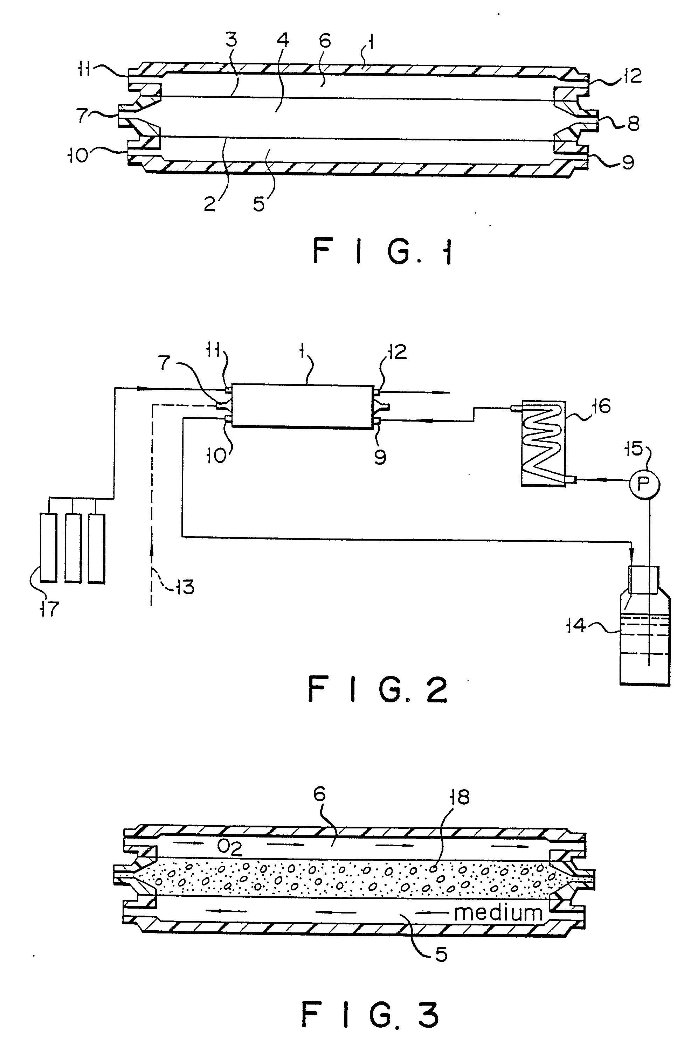

Fig. 1 is a cross-sectional view of one embodiment of a cell-incubator according to the invention. In Fig. 1, reference numeral 1 denotes a housing formed of synthetic resin. Inside the housing 1, substance exchanging membranes 2 and 3 are transversally stretched, and thus a room inside the housing 1 is divided into three compartments. The center compartment is a cell culture chamber 4. A compartment under the chamber 4 is a liquid fluid channel 5, and another compartment above the chamber 4 is a gas fluid channel 6. The housing 1 has several inlet ports and outlet ports provided on the side walls thereof. Ports 7 and 8 are for charging cells and communicated with the cell culture chamber 4. Ports 9 and 10 are inlet port and outlet port respectively for liquid medium, both of which are communicated with the liquid fluid channel 5. Ports 11 and 12 are inlet port and outlet port respectively for incubation gas, both of which are communicated with the gas fluid channel 6.

Fig. 2 shows a method to use the cell-incubator described above. First, cells to be cultured are charged

3

into the culture chamber 4. At this time, cells are preferably contained in a suspension which is supplied to the cell-charging port 7 through the line 13. A culture solution stored in a tank 14 is circulated through the liquid fluid channel 5 by a pump 15. At this time, a heat exchanger 16 is preferably provided in the circuit to adjust the temperature of the culture solution. While, through the gas fluid channel 6, an incubation gas prepared to include given ingredients of gases (e.g. gas containing $O_2$) is flowed by a pomb 17. By above procedures, the conditions shown in Fig. 3 are obtained in the inside of the cell-incubator. As shown in the figure, the culture chamber 4 is supplied with cells 18 to be cultured, the liquid fluid channel 5 is with a culture solution, and the gas fluid channel 6 is with incubation gas containing $O_2$. From the culture solution to the cells in the culture chamber 4, solutes such as nutrition ingredients which are necessary for cell-culturing are supplied through the substance exchanging membrane 2. Also, from incubation gas flowing in the gas channel 6 to the cells 18, $O_2$ which is necessary for cell-culturing is supplied through the substance exchanging membrane 3. As a result, desirable culture conditions can be maintained in the culture chamber 4 to promote cell-culturing.

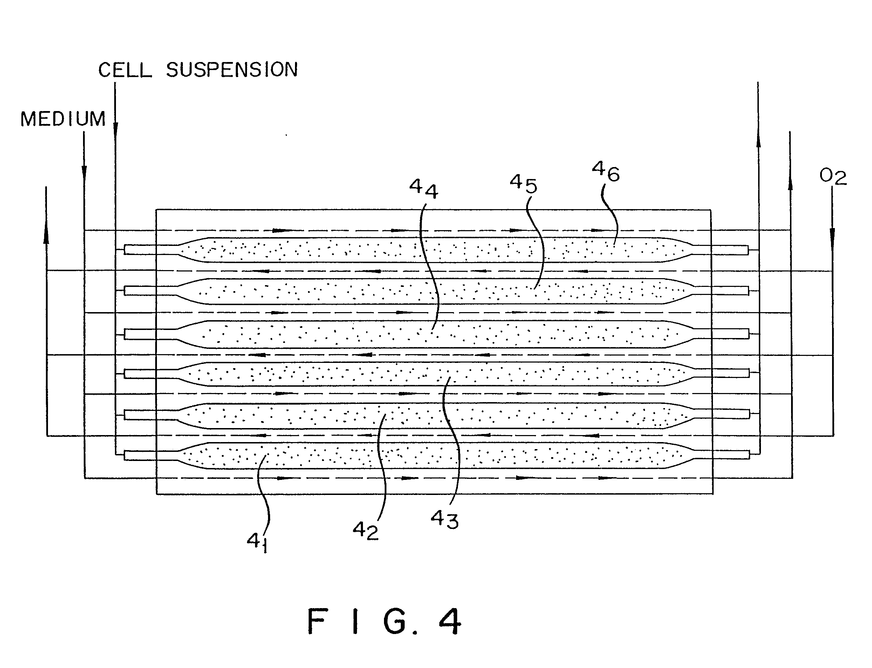

Fig. 4 shows another embodiment of a cell-incubator of the present invention. For this embodiment, a plural of culture chambers 41-46 are stacked. Each chamber is separated by either a liquid fluid channel or a gas fluid channel. As shown in this embodiment, the cell-incubator of the present invention can be realized also in such a form in which any number of culture chambers are stacked.

For both embodiments shown in Figs. 1 and 4, the first medium fluid channel is used for flowing a culture solution, and the second medium fluid channel is used for incubation gas. However, if circumstances require, both of the first and second medium fluid channels may be used for flowing a culture solution, and both channels are also used for incubation gas. In other words, culture solutions of two kinds can be flowed, or incubation gases of two kinds can be supplied.

The following is description of examples with reference to manufacturing and use of a cell-incubator of the invention.

Example 1 (production of a cell-incubator)

A stock solution for forming a film comprising polypropylene, liquid paraffin and additives was extruded from a T-die and cooled in a liquid paraffin solution, thereby being cured. The resultant solid was treated with a solvent to extract liquid paraffin and was thermally cured. A hydrophobic porous membrane A is thus obtained, which has no skin layer and is 0.45 μm in mean hole diameter and 80 μm in membrane thickness.

Also, a hydrophobic porous membrane B was obtained, which is 0.05 μm in mean hole diameter and is 130 μm in membrane thickness by the same procedure as described above.

Next, the obtained hydrophobic porous membrane A was punched out into a sheet of predetermined shape. This sheet of membrane A was disposed in a reaction container (30 mm in inner diameter) of a plasma treatment machine, such that the sheet was not contacted with the side-wall of the container. Then plasma was generated by applying 10 W power to induction coils which was connected to a high-frequency generator of 13.56 MHz under the condition that Ar gas is at 0.1 Torr, in order to irradiate the hydrophobic porous membrane A with the plasma for ten seconds. After the plasma irradiation, N,N-dimethylacrylamide gas was introduced in the reaction container, and graft reaction was performed at 20°C and 1 Torr for ten minutes. The obtained membrane was washed with methanol in Soxhlet extraction apparatus for 50 hours and dried under reduced pressure. The membrane thus treated was found completely hydrophilic including the inner region of the membrane.

The membrane A' which was made to hydrophilic as described above and the hydrophobic membrane B were welded, such that between the membranes an inlet port member and a outlet port member made of polypropylene were mounted, to produce a construction of a wrapper form. This construction was fixed in a molded form of housing as shown in Fig. 1 to form a cell-incubator as shown for Fig. 1.

Example 2 (cell culture)

From Sprague-Dawley series rats of 5-8 weeks (male), hepatocytes were separated according to Seglen method, etc. $4 \times 10^7$ of the hepatocytes were mixed with $2 \times 10^7$ fibroblast cells (passage number: 2-8) which was obtained by separating lungs of newborn rats (male) of 3 days of the same kind as said-above by collagenase-treatment. The cells obtained were suspended in a solution of 50 mℓ containing 0.2% of collagen. A culture solution was prepared by adding 0.4 μg/mℓ of hydrocortisone, 5 μg/mℓ of insulin, 5

4

$\mu$g/m$\ell$ of transferrin , 2 x 10$^{-9}$ M triiodthyronine, 10$^{10}$ M cholera toxine, 10$^{-4}$ M adenin, 10 ng/m$\ell$ of EGF, and 10% fetal bovine serum into Dulbecco-modified Eagle's medium (DEM). This solution was cooled to 4°C at use.

The cell suspension obtained above was charged into the culture chamber 4 as described in reference to Fig. 2. Also, the culture solution heated to 37°C and incubation gas containing 40% of $O_2$, 5% of $CO_2$, and 55% of $N_2$ were supplied into the cell-incubator as described in reference to Fig. 2. Cell culture was thus performed in a clean bench.

As observed on the seventh day of culturing, the hepatocytes formed a network construction through all the gel to give intercellular bile duct structure.

Example 3 (use as a artificial liver apparatus)

The cell-incubator in which the hepatocytes had been cultured at high density according to the Example 2 was used as an artificial liver apparatus. By utilizing this artificial liver apparatus, detoxicating experiment was conducted for simulated patient's blood as follows.

Specifically, the blood was poured into the container 14 of Fig. 2, and supplied to the liquid fluid channel 5 by means of the pump 15 for circulation. At this time, the blood was stirred by utilizing a magnetic stirrer.

Ingredients of the simulated patient's blood used and the circulating conditions are as follows;

[the simulated patient's blood]

heparinized bovine blood (heparin: 3 units/m$\ell$); 500 m$\ell$
hematocrit value; 35%
ammonium chloride; 500 $\mu$g/d$\ell$

[circulating conditions]

a flow rate of the simulated patient's blood; 100 m$\ell$/minute
a flow rate of oxygen; 150 m$\ell$/minutes

In this experiment, ammonium chloride concentration and urea concentration in the blood was measured with time. The results are shown in the following Table 1.

Table 1

| | Ammonium ($\mu$g/dl) | Urea (mg/d$\ell$) |
|---|---|---|
| before circulation | 530 | 15 |
| 30 min. after circulation | 500 | 17 |
| 60 min. after circulation | 480 | 19 |
| 120 min. after circulation | 450 | 22 |
| 180 min. after circulation | 420 | 25 |

As shown in Table 1, this artificial liver enables activities of the hepatocytes to maintain high. The apparatus was thus useful as an artificial liver.

As described above in detail, a cell-incubator of the invention is suitable for cultivating high-functional cells of high adhesion dependency and oxygen-requiring cells, and which easily gives appropriate culture conditions.

Also, the apparatus of the invention in which hepatocytes has already cultured at high-density can be used as an artificial liver without any modification. Upon using the artificial liver apparatus, activities of the hepatocytes can be maintained high and stable for a long period. Therefore, blood clearance can be continuously performed for a long time.

Claims

1. A cell-incubator comprising;
a housing (1) which has a room defined by housing walls,
an inlet port (9) and an outlet port (10) for a first medium, both of the ports being provided on said housing wall,
an inlet port (11) and an outlet port (12) for a second medium, both of the ports being provided on said housing wall,
a first medium fluid channel (5) formed in said room of the housing, the channel communicating with the inlet port and the inlet port for the first medium,
a second medium fluid channel (6) formed in said room of the housing, the channel communicating with the inlet port and the outlet port for the second medium,
a first substance exchanging membrane (2) which constitutes at least a part of a wall defining said first medium fluid channel,
a second substance exchanging membrane (3) which constitutes at least a part of a wall defining said second medium fluid channel, and
a culture chamber (4) which is formed between said first medium fluid channel and said second medium fluid channel, at least a part of walls defining said culture chamber being said first and second substance exchanging membrane. ,

2. A cell-incubator according to claim 1, characterized in that at least one of said first medium fluid channel and said second medium fluid channel is a liquid fluid channel for flowing a liquid medium, and a substance exchanging membrane which constitutes at least a part of a wall determining said liquid channel is a hydrophilic porous membrane.

3. A cell-incubator according to claim 1, characterized in that at least one of said first medium fluid channel and said second medium fluid channel is a gas fluid channel for flowing a gaseous medium, and a substance exchanging membrane which constitutes at least a part of a wall determining said gas channel is a hydrophobic porous membrane.

4. A cell-incubator according to claim 1, characterized in that said first medium fluid channel is for flowing a liquid medium and said second medium fluid channel is for flowing a gaseous medium.

5. A cell-incubator according to any one of claims 1 to 4, characterized by further comprising at least one selected from a group consisting of a porous sponge sheet having open pore, a mesh and collagen gel, all of which being charged into said culture chamber.

6. A cell-incubator according to any one of claims 1 to 5, characterized by further comprising a charging port (7) for cells, the port being provided in said housing and communicated with said culture chamber.

7. A cell-incubator according to any one of claims 1 to 6, characterized in that a plural of said culture chambers (4₁ to 4₆) are stacked, and between the stacked chambers said first medium fluid channel and said second medium fluid channel are provided alternately. 8. An artificial liver apparatus comprising;
a housing (1) which has a room defined by housing walls,
an inlet port (9) and an outlet (10) port for flowing blood, both of the ports being provided on said housing wall,
an inlet port (11) and an outlet port (12) for flowing an oxygen-containing gas, both of the ports being provided on said housing wall,
a blood channel (5) formed in said room of the housing, the channel communicating with said inlet port and said outlet port for flowing blood,
a gas channel (6) formed in said room of the housing, the channel communicating with said inlet port and said outlet port for flowing said oxygen-containing gas,
a substance exchanging membrane (2) which constitutes at least a part of a wall defining said blood channel,
a gas exchanging membrane (3) which constitutes at least a part of a wall defining said gas channel for an oxygen-containing gas,
a chamber (4) for containing hepatocytes, the chamber being formed between said blood channel and said gas channel, and at least a part of a wall defining said chamber being said substance exchanging membrane and said gas exchanging membrane, and
hepatocytes (18) which are charged in said chamber such that they are enclosed in collagen gel.

9. An artificial liver apparatus according to claim 8, characterized by further comprising a port (7 8) for charging hepatocytes, the port being provided on said housing wall and communicated with said chamber for the cells.

6

10. An artificial liver apparatus according to claim 8 or 9, characterized in that a plural of said chambers ($4_1$ to $4_6$) for hepatocytes are stacked, and between the stacked chambers said blood channel and said gas channel are provided alternately.

# F I G. 1

# F I G. 2

# F I G. 3

CELL SUSPENSION

MEDIUM

$4_4$   $4_5$   $4_6$

$O_2$

$4_1$   $4_2$   $4_3$

F I G. 4

EP 0 363 262 A1

European Patent Office

**EUROPEAN SEARCH REPORT**

Application Number

EP 89 40 2692

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 112 155 (BIO-RESPONSE INC.) * Page 8, line 7 - page 9, line 5; page 12, line 12 - page 13, line 31; page 17, line 4 - page 21, line 14; page 25, line 1 - page 26, line 8; claims 6-13; figures 2,6,9,10,16 * | 1-10 | C 12 M   3/00 |
| X | FR-A-2 393 849 (BATTELLE-INSTITUT E.V.) * Page 4, line 19 - page 6, line 15; example 1; figures 1-3 * | 1-10 | |
| X | US-A-4 446 229 (R.B. INDECH) * Whole document * | 1-10 | |
| P,X | WO-A-8 900 188 (BRUNSWICK CORP.) * Page 23, line 23 - page 35, line 9; claims 1-8; figures 3-7 * | 1-10 | |
| A | CHEMICAL ABSTRACTS, vol. 109, 1988, page 387, abstract no. 236960h, Columbus, Ohio, US; S. KASAI et al.: "Studies of hybrid artificial liver. Application of hepatocytes culture substrata for metabolic culture system", & JINKO ZOKI 1988, 17(1), 213-16 | 8-10 | TECHNICAL FIELDS SEARCHED (Int. Cl.5)  C 12 M |

The present search report has been drawn up for all claims

| Place of search | Date of completion of the search | Examiner |
|---|---|---|
| THE HAGUE | 21-12-1989 | GROENENDIJK M.S.M. |